# EUROPEAN PATENT APPLICATION

(11) **EP 4 431 179 A1**
(43) Date of publication of application: **18.09.2024**
(21) Application number: 24163905.3
(22) Date of filing: 15.03.2024
(51) Int. Cl.: B01J 20/10, B01J 20/32, B01J 20/28, B01J 20/287, B01D 15/32

(54) **A NOVEL STATIONARY PHASE FOR LARGE SCALE REVERSE-PHASE HPLC PURIFICATION**

(30) Priority: 17.03.2023 EP 23162706
(71) Applicant: Nouryon Chemicals International B.V., 1101 BZ Amsterdam (NL)
(72) Inventor: LE BINH TRAN, Therése, 1101 BZ Amsterdam (NL); SIGVARD PESSON, Börje, 1101 BZ Amsterdam (NL); HÖGBLOM, Joakim Erik Patrik, 1101 BZ Amsterdam (NL)
(74) Representative: LKGlobal UK Ltd.

(57) **Abstract**

The invention relates to silica particles having a fine pore size of 1 to 250 Angstrom (Å) and comprising a silane group which comprises two groups which are each independently selected from the group consisting of alkyl, aryl, alkylaryl, heteroalkyl, heteroaryl, and heteroalkylaryl. The present invention further relates to a method of preparing such silica particles as well as the use of such silica particles as stationary phase for purifying a modified conjugated peptide, such as a GLP-1 agonist or a GLP-2 analog.

## Description

### Technical Field

The invention relates to silica particles having a fine pore size of 1 to 250 Angstrom (Å) and comprising a silane group which comprises two groups which are each independently selected from the group consisting of alkyl, aryl, alkylaryl, heteroalkyl, heteroaryl, and heteroalkylaryl. The present invention further relates to a method of preparing such silica particles as well as the use of such silica particles as stationary phase for purifying a modified conjugated peptide, such as a GLP-1 agonist or a GLP-2 analog.

### Background Art

Reverse phase HPLC (high performance liquid chromatography) is a commonly used technique to separate, identify, and quantify all components which are contained in a given mixture. In the pharmaceutical field HPLC is particularly suitable for achieving the required high-grade purification of medicaments, in particular medicaments which have a higher molecular weight, such as modified conjugated peptides. The basic principle of HPLC is that the mixture to be purified is pumped together with a liquid solvent under pressure through a column filled with a solid adsorbent material, the so-called stationary phase, which is commonly a silica-based material. Each component of the mixture to be separated interacts slightly different with the adsorbent material usually due to more or less pronounced physical-chemical interaction of the components with the absorbent material's surface and/or the pore size of the absorbent material. This interaction causes different flow rates for the different components and results in a separation of the mixture components. The different components can then be collected in different fractions when they flow out from the column.

There are many different adsorbent materials for HPLC columns available. Particular attention has been drawn to silica particles which have been found to be very suitable from view of separation result, costs and raw material availability. A particular focus when optimizing these silica particles for HPLC use has been put on the particle size as well as on the pore sizes of the particles. However, there have also been made some efforts to modify the silica particles with functional groups to fine-tune the physical-chemical interaction of the absorbent material in the stationary phase with any potential component in a mixture to be separated. For instance, US 9,670,067 B2 describes the modification of silica particles with an organic group comprising a halohydrin group. Likewise, US 10,690,6365 B2 discloses silica particles modified with C8 or C18 groups. In US 7,537,703 B2, silica particles have been modified with n-butyl groups.

Modified conjugated peptides have been found to be challenging as regards purification thereof. It is assumed that this is due to their relatively high molecular weight which makes it tedious to separate a desired peptide from impurity peptides of somewhat similar molecular weight. For the purification of such conjugated peptides usually more than one purification step is necessary. However, from a commercial point of view, the number of purification steps should be ideally limited to only two with at the same time achieving commercially acceptable yields of highly purified conjugated peptide. In the pharmaceutical area, it is usually targeted at purities, i.e. selectivity, of at least >99.5%. Hence, a commercially suitable silica for the stationary phase should be suitable to achieve separation of the component of a mixture into ideally only fractions containing one pure component (i.e. with a purity of at least >99.5%) so that at the same high selectivity as well as high recovery, i.e. overall yield, of this one pure compound is achieved. In other words, for a commercially suitable stationary HPLC phase, it is not sufficient that maybe some fractions contain a compound with high purity. Rather, also the overall yield of this compound with high purity is an important factor. Commercially relevant overall yields are for instance at least 70%; preferably at least 75%, and even more preferably at least 80%.

Examples of conjugated peptides which are of pharmaceutical interest are for instance GLP-1 (glucagon-like peptide 1) receptor agonists and GLP-2 (glucagon-like peptide 2) analogs. These types of conjugated peptides are used in obesity treatment. A commercial relevant GLP-1 receptor agonist is for instance semaglutide which is an antidiabetic medicament used for the treatment of type 2 diabetes and as anti-obesity medication for long-term weight management. Another commercial relevant GLP-1 receptor agonist is liraglutide (N-ε-(γ-Glu(N-α-hexadecanoyl)))-Lys²⁶Arg³⁴-GLP-1(7-37), also known as NN2211. Liraglutide has been approved for the treatment of type 2 diabetes and for the treatment of obesity in adults.

However, in addition to the already above described generally encountered problems when purifying conjugated peptides, the purification of glucagon-like peptides has been found to be particularly demanding due to their tendency to form aggregates in particular at acidic pH.

In summary, there is still a need for improved stationary phases for HPLC applications, in particular for purification of medicaments, such as high-molecular weight molecules, for instance modified conjugated peptides like GLP-1 receptor agonists and GLP-2 analogs, in particular for liraglutide and semaglutide. In particular, there is a need for improved silica particles for HPLC applications which have improved separation performance as regards selectivity (i.e. purity) and recovery (i.e. yield), and which, at the same time, are ideally easily accessible, readily available, and environmentally harmless. Moreover, there is also a need to provide methods for preparing such improved silica particles which can be used in HPLC application for successful and efficient purification of modified conjugated peptides like GLP-1 receptor agonists and GLP-2 analogs.

The present invention is, therefore, directed to finding such improved silica particles and methods for producing the same. It has been found that the above problems can be solved by improved silica particles having a fine pore size of 1 to 250 Angstrom (Å) and comprising a silane group which comprises two groups which are each independently selected from the group consisting of alkyl, aryl, alkylaryl, heteroalkyl, heteroaryl, and heteroalkylaryl.

### Summary of Invention

In a first aspect, the present invention is directed to silica particles having a fine pore size of 1 to 250 Angstrom (Å) and comprising a silane group according to the following formula: 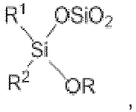 wherein R¹ and R² are each independently selected from the group consisting of alkyl, aryl, alkylaryl, heteroalkyl, heteroaryl, and heteroalkylaryl; R is hydrogen or silica; and SiO₂ is silica.

In a second aspect, the present invention is further directed to a method for preparing a silica particle comprising the following steps:
(i) providing a silica particle with a fine pore size of 1 to 250 Angstrom (Å); and
(ii) modifying the silica particle with a compound of the following formula 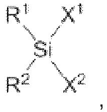
wherein R¹ and R² are each independently selected from the group consisting of alkyl, aryl, alkylaryl, heteroalkyl, heteroaryl, and heteroalkylaryl; and X¹ and X² are independently a halogen or an alkoxy group.

The present invention is even further directed to the use of the silica particle according to the first aspect or a silica particle obtained by the method according to the second aspect as stationary phase for purifying a modified conjugated peptide, preferably a GLP-1 agonist or a GLP-2 analog.

### Brief Description of Drawings

Figure 1a shows a chromatogram obtained for the first purification step of example 2 a).
Figure 1b shows a chromatogram obtained for the second purification step of example 2 a).
Figure 2a shows a chromatogram obtained for the first purification step of example 2 b).
Figure 2b shows a chromatogram obtained for the second purification step of example 2 b).
Figure 3a shows a chromatogram obtained for the first purification step of example 3.
Figure 3b shows a chromatogram obtained for the second purification step of example 3.

### Description of Embodiments

In a first aspect, the present invention discloses novel stationary phases for large scale reversed phase HPLC purification, in particular for purification of modified conjugated peptides, such as GLP-1 receptor agonists and GLP-2 analogs. In particular, the present invention provides a silica particle which can be used as a stationary phase for large scale reversed phase HPLC purification.

The novel stationary phases according to the present invention comprise a silica particle having a fine pore size of 1 to 250 Angstrom (Å) and comprising a silane group according to the following formula: 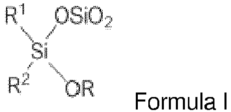 wherein R¹ and R² are each independently selected from the group consisting of alkyl, aryl, alkylaryl, heteroalkyl, heteroaryl, and heteroalkylaryl; R is hydrogen or silica; and SiO₂ is silica.

Surprisingly, it has been found that such modified silica particle can be used for a stationary phase in HPLC application and achieves improved separation characteristics in particular for modified conjugated peptides, such as modified conjugated peptides, for instance GLP-1 receptor agonists and GLP-2 analogs, and in particular for liraglutide and semaglutide. With these new modified silica particles in stationary phase for HPLC applications, specific difficult impurities in crude active pharmaceutical ingredients, such as in liraglutide and semaglutide, can be successfully removed already in a first chromatographic step, for instance under alkaline conditions. This allows to deliver an already more purified active pharmaceutical ingredient per cycle and hour to the second chromatographic step which in turn increases the possibility to meet the pharmaceutical industry's purity demands of >99.5% and single impurity amount of <0.1% already in a second HPLC purification step with, at the same time, commercially acceptable overall yields. In other words, the modified silica particle of the present invention increases the selectivity as well as the recovery significantly, resulting in an overall significantly improved separation productivity.

In the above Formula I, the rests R¹ and R² are each independently selected from the group consisting of alkyl, aryl, alkylaryl, heteroalkyl, heteroaryl, and heteroalkylaryl.

The term "alkyl" refers to a straight, branched chain and/or cyclic ("cycloalkyl") hydrocarbon having from 1 to 30 (e.g. 1 to 20, or 1 to 4) carbon atoms. Alkyl moieties having from 1 to 4 carbons are referred to as "lower alkyl." Examples of alkyl moieties include methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl and dodecyl. Cycloalkyl moieties may be monocyclic or multicyclic, and examples include cyclopropyl, cyclobutyl, cyclopentyl, cyclohexyl and adamantyl. Additional examples of alkyl moieties have linear, branched and/or cyclic portions (e.g. 1-ethyl-4-methyl-cyclohexyl). The term "alkyl" as used herein includes saturated as well as unsaturated hydrocarbons, preferably saturated hydrocarbons.

The term "aryl" refers to an aromatic ring or an aromatic or partially aromatic ring system composed of carbon and hydrogen atoms. An aryl moiety may comprise multiple rings bound or fused together. Examples of aryl moieties include anthracenyl, azulenyl, biphenyl, fluorenyl, indan, indenyl, naphthyl, phenanthrenyl, phenyl, 1,2,3,4-tetrahydro-naphthalene and tolyl.

The term "alkylaryl" refers to a moiety comprising at least one alkyl group and at least one aryl group, such as an alkyl moiety bound to an aryl moiety, wherein the terms "alkyl" and "aryl" are as defined above.

The term "heteroalkyl" refers to an alkyl group which also contain at least one heteroatom, such as a nitrogen, oxygen, phosphor, sulfur and/or boron atom, wherein the term "alkyl" is as defined above.

The term "heteroaryl" refers to an aryl group which also contains at least one heteroatom, such as a nitrogen, oxygen, phosphor, sulfur and/or boron atom, wherein the term "aryl" is as defined above. For instance, the term "heteroaryl" preferably refers to an aryl group wherein at least one of its carbon atoms has been replaced with nitrogen, oxygen, or sulfur. Examples of heteroaryl moieties include acridinyl, benzimidazolyl, benzofuranyl, benzoisothiazolyl, benzoisoxazolyl, benzoquinazolinyl, benzothiazolyl, benzoxazolyl, furyl, imidazolyl, indolyl, isothiazolyl, isoxazolyl, oxadiazolyl, oxazolyl, phthalazinyl, pyrazinyl, pyrazolyl, pyridazinyl, pyridyl, pyrimidinyl, pyrimidyl, pyrrolyl, quinazolinyl, quinolinyl, tetrazolyl, thiazolyl, and triazinyl.

The term "heteroalkylaryl" refers to a moiety comprising at least one alkyl group, at least one aryl group, and at least one heteroatom, such as a nitrogen, oxygen, phosphor, sulfur and/or boron atom, wherein the terms "alkyl" and "aryl" are as defined above.

Any of the above groups alkyl, aryl, alkylaryl, heteroalkyl, heteroaryl, and heteroalkylaryl may be unsubstituted or may be further substituted.

The term "substituted," when used to describe a chemical structure or moiety, refers to a derivative of that structure or moiety wherein one or more of its hydrogen atoms is substituted with an atom, chemical moiety or functional moiety such as, but not limited to, alcohol, aldehylde, alkoxy, alkanoyloxy, alkoxycarbonyl, alkenyl, alkyl (e.g., methyl, ethyl, propyl, t-butyl), alkynyl, alkylcarbonyloxy (-OC(O)alkyl), amide (-C(O)NH-alkyl- or -alkyl-NHC(O)alkyl), amidinyl (-C(NH)NH-alkyl or -C(NR)NH2), amine (primary, secondary and tertiary such as alkylamino, arylamino, arylalkylamino), aroyl, aryl, aryloxy, azo, carbamoyl (-NHC(O)O-alkyl- or -OC(O)NH-alkyl), carbamyl (e.g. CONH₂, as well as CONH-alkyl, CONH-aryl, and CONH-arylalkyl), carbonyl, carboxyl, carboxylic acid, carboxylic acid anhydride, carboxylic acid chloride, cyano, ester, epoxide, ether (e.g. methoxy, ethoxy), guanidino, halo, haloalkyl (e.g. -CCl₃, -CF₃, -C(CF₃)₃), heteroalkyl, hemiacetal, imine (primary and secondary), ketone, nitrile, nitro, oxygen (i.e., to provide an oxo moiety), phosphodiester, sulfide, sulfonamido (e.g. SO₂NH₂), sulfone, sulfonyl (including alkylsulfonyl, arylsulfonyl and arylalkylsulfonyl), sulfoxide, thiol (e.g. sulfhydryl, thioether) and urea (-NHCONH-alkyl-).

In some embodiments, R¹ and R² contain both independently 2 to 20 carbon atoms
In some embodiments, R¹ and R² are preferably both independently selected from the group consisting of alkyl and heteroalkyl. In some embodiments, R¹ and R² are both independently alkyl, such as C₂-C₂₀ branched or unbranched alkyl, preferably C₂-C₁₀-branched or unbranched alkyl, such as C₃-C₁₀-branched or unbranched alkyl or C₃-C₈-branched or unbranched alkyl, and even more preferably C₄-C₈-branched or unbranched alkyl, such as C₄-branched or unbranched alkyl, C₅-branched or unbranched alkyl, C₆-branched or unbranched alkyl, C₇-branched or unbranched alkyl, or C₈-branched or unbranched alkyl, or for instance C₄-C₆-branched or unbranched alkyl. Particular preferred are unbranched alkyls, such as C₂-C₂₀-unbranched alkyl, C₂-C₁₀-unbranched alkyl, C₃-C₁₀-unbranched alkyl, C₃-C₈-unbranched alkyl, or even C₄-C₈-unbranched alkyl, such as C₄-unbranched alkyl, C₅-unbranched alkyl, C₆-unbranched alkyl, C₇-unbranched alkyl, or C₈-unbranched alkyl, or for instance C₄-C₆-unbranched alkyl. In a particularly preferred embodiment, R¹ and R² are both independently C₄-, C₆- and/or C₈-branched or unbranched alkyl, and even more preferred C₄-, C₆- and/or C₈-unbranched alkyl. In another preferred embodiment, R¹ and R² are both the same. For instance, in some embodiments R¹ and R² are both C₄-, C₆- or C₈-branched or unbranched alkyl, such as C₄- or C₈-branched or unbranched alkyl, and even more preferred C₄-, C₆- or C₈-unbranched alkyl, such as C₄- or C₈-unbranched alkyl.

"SiO₂" in the above Formula I means silica. This means the silane group of Formula I is bond to a silica structure, i.e. a silica particle, via a O-Si-bond.

The silica or particle of silica may be obtained from e.g. precipitated silica, micro silica (silica fume), pyrogenic silica (fumed silica), silica sols or silica gels, and mixtures thereof. The particle of silica may be in the form of a porous particle.

Suitably, the particle of silica has a specific surface area from 20 to 1500, preferably from 50 to 900, and most preferably from 70 to 800 m²/g. The specific surface area can be measured by means of titration with sodium hydroxide as described by Sears in Analytical Chemistry 28(1956), 12, 1981-1983 and in U.S. Pat. No. 5,176,891. The given area thus represents the average specific surface area of the particles.

The rest R in the above formula is either hydrogen or silica. "Silica" means in this context that the silane group of Formula I is bond to the silica or silica particle via a further O-Si-bond.

The silica particle of the present invention has a fine pore size of 1 to 250 Angstrom (Á), such as for instance from 10 to 200 Å, or from 50 to 250 Å, or from 60 to 200 Å, or even from 70 to 180 Å. Particularly preferred are fine pore sizes of 80 to 150 Å, such as from 100 to 130 Å, and in particular a fine pore size of around 100 Å or around 120 Å. It has been found that fine pore sizes above 250 Å tend to significantly impair the overall separation productivity of the silica particle in a HPLC column, i.e. the overall yield of purified components is low. All references to a fine pore size as used herein refer to the average fine pore size. Methods for determining the fine pore size of a silica particle are commonly known by the skilled person and include for instance the Brunauer-Emmett-Teller (BET)-method by for instance using the device TriStar II Plus from Micromeritics.

The particle size of the silica particle of the present invention is not particularly limited. For instance, the silica particle of the present invention may have an average particle diameter of 0.1 to 100 µm, and preferably from 1 to 50 µm. In some embodiments, it may even have an average particle diameter ranging from 1.8 to 25 µm. Methods for determining the average particle diameter of a silica particle are commonly known by the skilled person. For instance, the particle diameter may be calculated from the formula relating to specific surface area and particle diameter in Iler (The Chemistry of Silica, Wiley, 1979). As conventional in silica chemistry, the particle size refers to the average size of the primary particles. The particle size may also be measured via the coulter principle on a Multisizer 4e Coulter Counter from Beckman Coulter.

The pore volume of the particle of modified silica is suitably from 0.1 to 4 ml/g, preferably from 0.2 to 2 ml/g, most preferably from 0.3 to 1.2 ml/g.

The specific surface area (BET method) of the particle of modified silica containing is suitably from 1 to 1000 m²/g, preferably from 25 to 700 m²/g, most preferably from 50 to 500 m²/g.

A suitable way of measuring surface area, pore volume and average fine pore size (from the surface area and pore volume) is by the Brunauer-Emmett-Teller (BET) method, also based on nitrogen adsorption/desorption, the surface area typically being calculated from the linear part of the isotherm. Examples of such methods are given in ISO9277:2010 (for BET) and ISO 15901-2:2006 (for gas adsorption/desorption). Preferably, surface area, pore volume and average fine pore size are measured using ISO9277:2010.

In another aspect, the present invention describes a method for preparing a silica particle comprising the following steps:
(i) providing a silica particle with a fine pore size of 1 to 250 Angstrom (Å); and
(ii) modifying the silica particle with a compound of the following formula

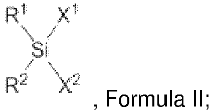

wherein R¹ and R² are each independently selected from the group consisting of alkyl, aryl, alkylaryl, heteroalkyl, heteroaryl, and heteroalkylaryl; and
X¹ and X² are each independently a halogen or an alkoxy group.

With this method it is possible to obtain a silica particle according to the first aspect of the present invention as described above. Hence, the present invention is also directed to silica particles obtained by the method as described above according to this aspect of the present invention.

The silica particle provided in step (i) of the method according to the second aspect of the present invention may be as described above and may have the same preferred fine pore sizes as described above in the context of the first aspect of the present invention.

The compound according to Formula II which is used in step (i)i of the method according to the second aspect of the present invention to modify the silica particle is a silane which comprises two groups R¹ and R² which are each independently selected from the group consisting of alkyl, aryl, alkylaryl, heteroalkyl, heteroaryl, and heteroalkylaryl, and two further groups X¹ and X² which are each independently a halogen or an alkoxy group.

Groups R¹ and R² may be the same as described above in the context of the first aspect of the present invention.

Groups X¹ and X² which are each independently a halogen or an alkoxy group, preferably a halogen.

Halogens include fluorine, chlorine, bromine and iodine, of which chlorine is particularly preferred.

The term "alkoxy group" refers to an -O-alkyl moiety, wherein the alkyl group can be as defines above in the context of the first aspect of the present invention. Preferred are alkoxy groups wherein the alkyl moiety has 1-8 carbon atoms, such as 1-4 carbon atoms. Examples of alkoxy moieties include -OCH₃, -OCH₂CH₃, -O(CH₂)₂CH₃, -O(CH₂)₃CH₃, - O(CH₂)₄CH₃, and -O(CH₂)₅CH₃.

The modification reaction as such is known by the skilled person and can be adjusted according to needs. For instance, the reaction of the compound of Formula II with the silica particle is performed at a temperature suitable for performing the reaction, preferably from 40 to 180, more preferably from 60 to 160, and most preferably from 80 to 140° C.

Preferably, the compound of Formula II may be added to the silica particle under agitation and at a controlled rate, until a suitable amount of compound of Formula II has been added. The reaction time may be from 1 to 24 hours.

The reaction of the compound of Formula II and the silica particle may be carried out in an organic solvent, preferably under stirring. Such an organic solvent is preferably an aprotic solvent. Examples of such an aprotic solvent may be acetonitrile, acetone, xylene or toluene, preferably toluene, or mixtures thereof.

The silica particle may be added to the solvent, followed by an optional step of evaporating any water present. The compound of Formula II may then be added to a solvent, and thereafter added to the dispersion of silica particle and solvent.

The proportion of the compound of Formula II may be from 4 to 8 pmoles per m² particle of silica surface. The amount of solvent is preferably selected in such a way that the amount of silica particle in the dispersion is from 5 to 20 wt %.

The silanol surface moieties on the silica particle react with the compound of Formula II to form a covalent link between the silica and the compound of Formula II.

When the dispersion of modified silica particles has been formed, the dispersion may be cooled and purified, e.g. by ultra filtration, or by washing, e.g. by using toluene, ethanol or formic acid. The dispersion may then be dried, for example at 40 to 100° C., preferably at 60 to 90° C., for 2 to 30 hours, preferably from 10 to 25 hours.

According to an even further aspect, the present invention relates to a separation column for chromatography comprising the silica particle of the present invention, i.e. the silica particle according to the first aspect of the present invention or a silica particle obtained by the method according to the second aspect of the present invention. In this aspect, the silica particles of the invention have been packed into the separation column. Hence, in this aspect the silica particles of the invention are used a stationary phase for chromatography. The silica particles may thus be used in a separation column for any type of chromatographic separation methods, such as HPLC, supercritical fluid chromatography (SFC), and simulating moving bed (SMB).

According to an even further aspect, the present invention relates to the use of the silica particle according to the first aspect of the present invention or a silica particle obtained by the method according to the second aspect of the present invention as stationary phase for purifying a modified conjugated peptide, preferably a GLP-1 agonist or a GLP-2 analog. It is particularly preferred that the silica particle according to the first aspect of the present invention or a silica particle obtained by the method according to the second aspect of the present invention is used as stationary phase for purifying semaglutide or liraglutide.

### Examples

### Example 1: Preparation of modified silica particles

The following commercially available Kromasil^{®} particles (from Nouryon B.V.) were used as the starting material to prepare different modified silica particles.

Silica particles having an average particle size of 5 µm, an average pore size of 100 Å and a specific surface area of 315 m²/g (Kromasil^{®} KR-100-5 SIL of Nouryon B.V.).

Silica particles having an average particle size of 10 µm, an average pore size of 100 Å and a specific surface area of 320 m²/g (Kromasil^{®} KR-100-10 SIL of Nouryon B.V.).

Silica particles having an average particle size of 5 µm, an average pore size of 200 Å and a specific surface area of 195 m²/g (Kromasil^{®} KR-200-5 SIL of Nouryon B.V.).

Silica particles having an average particle size of 10 µm, an average pore size of 300 Å and a specific surface area of 105 m²/g (Kromasil^{®} KR-300-10 SIL of Nouryon B.V.).

### Example 1 a)

20 g of KR-100-5 SIL and 10.3 g imidazole was added to 175 g toluene. The resulting dispersion was heated to evaporate 20 g of toluene. After heating, the dispersion was cooled to 80 °C. 8 g of dibutyldichlorosilane was added to the dispersion of toluene, silica, and imidazole under stirring. The temperature was raised, and the dispersion was refluxed overnight. The reaction was cooled to 70° C and 32 g ethanol was added to deactivate the silane. The dispersion was washed by 87 g toluene and 2×79 g ethanol. The silica was dried at 90° C in a lab oven overnight. The resulting powder was white and is further referred to herein as Kromasil^{®} 100-5-diC4. Elemental analysis of carbon gave 8.3 wt % C.

### Example 1 b)

KR-100-10 SIL was modified in the same manner with dibutyldichlorosilane as described above in Example 1 a) for KR-100-5 SIL. The resulting powder was white and is further referred to herein as Kromasil^{®} 100-10-diC4. Elemental analysis of carbon gave 8.4 wt % C.

### Example 1 c)

KR-100-10 SIL was modified in the same manner as described above in Example 1 a) except of that 5.7 g butyldimethylchlorosilane and 4.1 g trimethylchlorosilane were added instead of dibutyldichlorosilane as surface modification. The resulting powder was white and is further referred to herein as Kromasil^{®} 100-10-C4. Elemental analysis of carbon gave 7.7 wt % C.

### Example 1 d)

KR-200-5 SIL was modified in the same manner with 5.0 g dibutyldichlorosilane, as described above in Example 1 a) for KR-100-5 SIL. The resulting powder was white and is further referred to herein as Kromasil^{®} 200-5-diC4. Elemental analysis of carbon gave 5.2 wt % C.

### Example 1 e)

KR-100-5 SIL was modified in the same manner as described above in Example 1 a) except of that instead of dibutyldichlorosilane, 12.4 g dioctyldichlorosilane was used. The resulting powder was white and is further referred to herein as Kromasil^{®} 100-5-diC8. Elemental analysis of carbon gave 14.8 wt % C.

### Example 1 f)

KR-100-5 SIL was modified in the same manner as described above in Example 1 a) except of that instead of dibutyldichlorosilane, 10.2 g dihexyldichlorosilane was used. The resulting powder was white and is further referred to herein as Kromasil^{®} 100-5-diC6. Elemental analysis of carbon gave 11.9 wt % C.

### Example 1 g)

KR-300-10 SIL was modified with 2.7 g dibutyldichlorosilane in the same manner as described above in Example 1a) for KR-100-5 SIL. The resulting powder was white and is further referred to herein as Kromasil^{®} 300-10-diC4. Elemental analysis of carbon gave 3.1 wt % C.

### Example 2: Two step purification of liraglutide

In all following examples, the samples to be purified was a mixture containing 5.0 mg mL⁻¹ of crude liraglutide (purity ≤ 10%) which was prepared by dissolving 80 mg of crude liraglutide in 16 mL of NH₄HCO₃ buffer (0.1 mol L⁻¹, pH 7.0) / Acetonitrile (95:5), filtered through a 0.45 µm syringe filter and which was injected directly in the column.

### Example 2 a): Two step purification of liraglutide on Kromasil@ 100-10-diC4

The first purification step was performed at pH 7 with ammonium hydrogen carbonate, and the second purification was performed at pH 8 with ammonium acetate as buffer, and in both systems, acetonitrile was used as the organic modifier. The purification was performed on an Agilent 1260 system equipped with an automated fraction collector. Both purification steps were made on a 4.6x250 mm column packed with Kromasil@ 100-10-diC4 from Example 1 b).

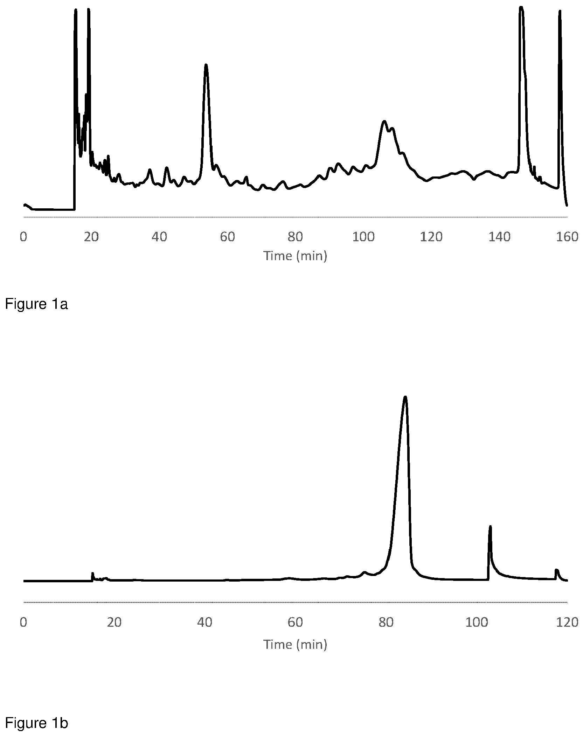

Frist purification step: A crude liraglutide sample (purity ≤ 10 %), was loaded onto the column, 32 mg/g stationary phase, where the mobile phase consisted of 0.1 M ammonium hydrogen carbonate, pH 7, and acetonitrile. The purification was performed under gradient elution, according to the following sequence. A loading step with 5 % acetonitrile for 5 minutes, between 5 minutes and 138 minutes, the acetonitrile concentration was increased linearly from 29.4 % to 39.8 %. After 128 minutes the acetonitrile concentration was increased to 80 % for 11 minutes to elute strongly adsorbed components and to wash the column, before the acetonitrile concentration was decreased to 5 % to equilibrate the column. Liraglutide was collected between 105 minutes and 115 minutes, and the pooled fractions showed a purity of 97.1 %, with a recovery of 81 %. The obtained chromatogram is shown in Figure 1a.

Second purification step: The pooled fractions from the first purification were diluted to about 10 % acetonitrile and loaded onto the column containing Kromasil@ 100-10-diC4, where the mobile phase consisted of 0.1 M ammonium acetate buffer at pH 8, and acetonitrile as organic modifier. The purification was performed under gradient elution, according to the following sequence A loading step with 5 % acetonitrile for 5 minutes, between 5 minutes and 95 minutes, the acetonitrile concentration was increased linearly from 30.5 % to 41.3 %. After 95 minutes the acetonitrile concentration was increased to 80 % for 11 minutes to elute strongly adsorbed components and to wash the column before the acetonitrile concentration was decreased to 5 % to equilibrate the column. Liraglutide was collected between 79 minutes and 86 minutes, and the pooled fractions showed a purity of ≥ 99.5 %, with recovery of 98.2%. Hence, the total recovery over both purification steps was 79.6 %. The obtained chromatogram is shown in Figure 1b.

### Example 2 b): Two step purification of liraglutide on Kromasil@ 100-5-diC8

The first purification step was performed at pH 7 with ammonium carbonate, and the second purification was performed at pH 8 with ammonium acetate as buffer, and in both systems, acetonitrile was used as the organic modifier. The purification was performed on an Agilent 1260 system equipped with an automated fraction collector. both purification steps were made on a 4.6x150 mm column packed with Kromasil@ 100-5-diC8 from Example 1 e) above.

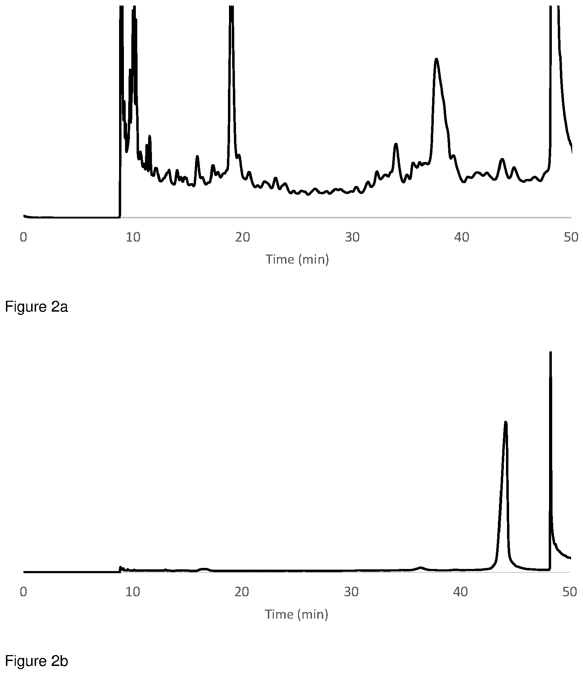

Frist purification step: A crude liraglutide sample (purity ≤ 10 %), was loaded onto the column, 12 mg/g stationary phase, where the mobile phase consisted of 0.1 M ammonium hydrogen carbonate, pH 7, and acetonitrile. The purification was performed under gradient elution, according to the following sequence. A loading step with 5 % acetonitrile for 5 minutes, between 5 minutes and 45 minutes, the acetonitrile concentration was increased linearly from 29.8 % to 40.2 %. After 45 minutes the acetonitrile concentration was increased to 70 % for 11 minutes to elute strongly adsorbed components and to wash the column before the acetonitrile concentration was decreased to 5 % to equilibrate the column. Liraglutide was collected between 37 minutes and 40 minutes, and the pooled fractions showed a purity of 96.4 %, with a recovery of 96 %. The obtained chromatogram is shown in Figure 2a.

Second purification step: The pooled fractions from the first purification was diluted to about 10 % acetonitrile, and loaded on to the column containing Kromasil@ 100-5-diC8, where the mobile phase consisted of 0.1 M ammonium acetate buffer at pH 8, and acetonitrile as organic modifier. The purification was performed under gradient elution, according to the following sequence. A loading step with 5 % acetonitrile for 5 minutes, between 5 minutes and 45 minutes, the acetonitrile concentration was increased linearly from 29.8 % to 40.2 %. After 45 minutes the acetonitrile concentration was increased to 70 % for 11 minutes to elute strongly adsorbed components and to wash the column before the acetonitrile concentration was decreased to 5 % to equilibrate the column. Liraglutide was collected between 43 minutes and 45 minutes, and the pooled fractions showed a purity of 99.6 %, with a total recovery over both purification steps of 79 %. The obtained chromatogram is shown in Figure 2b.

### Example 2 c): Two step purification of liraglutide on Kromasil^{®} 100-10-C4 (comparative example)

A two-step purification of a liraglutide sample on Kromasil^{®} 100-10-C4 from Example 1 c) above was performed in the same manner as described above in Example 2 a) for the two step purification of liraglutide on Kromasil@ 100-10-diC4 using the same solvent gradients and equipment parameters.

In the first purification step, the fractions were collected every minute between 100 and 125 minutes. Fractions No. 12 to 17 were pooled together for the second purification step. The pooled fractions showed a purity of 96.5 %, with a recovery of 87.4 %.

In the second purification step, the fractions were collected every 30 seconds, between 77 and 87 minutes. Pooled fractions No. 12 to 16 presented a purity of 99.5 %, with a recovery of 65.6 %. Hence, the total recovery over both purification steps was merely 57.3 %. This is considerably lower than the total recovery achieved for instance by using Kromasil@ 100-10-diC4 (see above Example 2 a)) for which a total recovery of 79.6 % was achieved..

### Example 2 d): Two step purification of liraglutide on Kromasil^{®} 300-10-diC4 (comparative example)

A two-step purification of a liraglutide sample on Kromasil^{®} 300-10-diC4 from Example 1 g) above was performed in the same manner as described above in Example 2 a) for the two-step purification of liraglutide on Kromasil@ 100-10-diC4 using the same solvent gradients and equipment parameters.

In the first purification step, the fractions were collected every minute between 92 and 109 minutes. Fractions No. 3 to 7 were pooled together for the second purification step. The pooled fractions showed a purity of 94.5 %, with a recovery of 73.8 %.

In the second purification step, the fractions were collected every 30 seconds, between 71 and 86 minutes. Only fraction No. 16 presented an acceptable purity of 99.5 %, with a recovery of 4.7 %. Hence, the total recovery over both purification steps was merely 3.4 %. This is considerably lower than the total recovery achieved for instance by using Kromasil@ 100-10-diC4 (see above Example 2 a)) for which a total recovery of 79.6 % was achieved.

### Example 3: Two step purification of semaglutide on Kromasil@ 100-10-diC4

The first purification step was performed at pH 7 with ammonium hydrogen carbonate, and the second purification was performed at pH 8 with ammonium acetate as buffer, and in both systems, acetonitrile was used as the organic modifier. The purification performed on an Agilent 1260 system equipped with an automated fraction collector. both purification steps were made on a 4.6x250 mm column packed with Kromasil@ 100-10-diC4 from Example 1 c) above. The semaglutide sample to be purified was a mixture containing 5.0 mg mL⁻¹ of crude semaglutide (purity ≤ 60%) which was prepared by dissolving 80 mg of semaglutide in 16 mL of NH₄HCO₃ buffer (0.1 mol L⁻¹, pH 7.0) / Acetonitrile (95:5), filtered through a 0.45 µm syringe filter and which was injected directly in the column

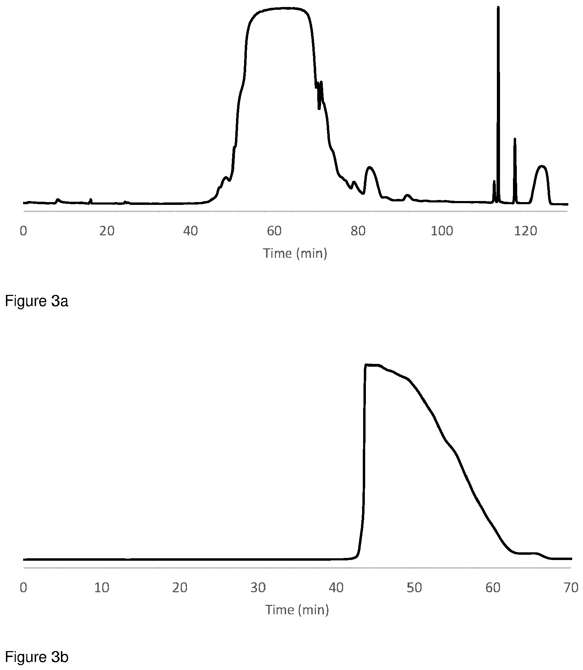

Frist purification step: A crude semaglutide sample (purity ≤ 60 %), was loaded on to the column, 34 mg/g stationary phase, where the mobile phase consisted of 0.1 M ammonium hydrogen carbonate, pH 7, and acetonitrile. The purification was performed under gradient elution, according to the following sequence. A loading step with 5 % acetonitrile for 5 minutes, between 5 minutes and 138 minutes, the acetonitrile concentration was increased linearly from 30% to 40.5 %. After 128 minutes the acetonitrile concentration was increased to 80 % for 11 minutes to elute strongly adsorbed components and to wash the column, before the acetonitrile concentration was decreased to 5 % to equilibrate the column. Semaglutide was collected between 50 minutes and 70 minutes, and the pooled fractions showed a purity of 98.1 %, with a recovery of 95 %. The obtained chromatogram is shown in Figure 3a.

Second purification step: The pooled fractions from the first purification was diluted to about 10 % acetonitrile, and loaded on to the column containing Kromasil@ 100-10-diC4, where the mobile phase consisted of 0.1 M ammonium acetate buffer at pH 8, and acetonitrile as organic modifier. The purification was performed under gradient elution, according to the following sequence. A loading step with 5 % acetonitrile for 5 minutes, between 5 minutes and 95 minutes, the acetonitrile concentration was increased linearly from 30.5 % to 41.3 %. After 95 minutes the acetonitrile concentration was increased to 80 % for 11 minutes to elute strongly adsorbed components and to wash the column before the acetonitrile concentration was decreased to 5 % to equilibrate the column. Liraglutide was collected between 40 minutes and 65 minutes, and the pooled fractions showed a purity of 99.6 %, with total a recovery over both purification steps of 76 %. The obtained chromatogram is shown in Figure 3b.

### Example 4: Further one step purification tests for liraglutide

Further experiments were made to confirm that various silica particles of the present invention are suitable to achieve already in a first purification step purity levels for liraglutide of at least 95 % with a commercially acceptable yield for a first purification step, such as at least 70 %, preferably at least 80 %, and more preferably at least 90 %. The liraglutide sample to be purified, the solvent gradient and the equipment parameters were the same as described above for Example 2 a). Merely the silica particles used in the HPLC column as stationary phase have been changed. In Example 4 a) Kromasil^{®} 100-5-diC4 from Example 1 a) above was used. In Example 4 b) Kromasil^{®} 200-5-diC4 from Example 1 d) above was used. In Example 4 c) Kromasil^{®} 100-5-diC6 from Example 1 f) above was used

Table 1 summarizes the results and also incorporates the results of Examples 2 and 3 above.

**Table 1: Liraglutide purification using different silica phases**

| Example | Silica phase | First purification step (%) | | Second purification step (%) | |
|---|---|---|---|---|---|
| | | Purity | Recovery | Purity | overall recovery * |
| 2 a) | 100-10-diC4 | 97.1 | 81 | ≥ 99.5 | 79.6 |
| 2 b) | 100-5-diC8 | 96.4 | 96 | 99.6 | 79 |
| 2 c) ⁺ | 100-10-C4 | 96.5 | 87.4 | 99.5 | 57.3 |
| 2 d) ⁺ | 300-10-diC4 | 94.5 | 73.8 | 99.5 | 3.4 |
| 3 ** | 100-10-diC4 | 98.1 | 95 | 99.6 | 76 |
| 4 a) | 100-5-diC4 | 97.8 | 90.1 | - | - |
| 4 b) | 200-5-diC4 | 96 | 70.5 | - | - |
| 4 c) | 100-5-diC6 | 95.6 | 96 | - | - |

| | | | | | |
|---|---|---|---|---|---|
| * = overall recovery over both purification steps ⁺ = comparative example ** = semaglutide was purified rather than liraglutide - = not performed | | | | | |

It is apparent from above Table 1 that the silica particles of the present invention are particularly suitable to achieve already in a first purification step purity rates of above 95 % with commercially acceptable yields of at least 70 %. Moreover, the silica particles of the present invention also achieve over all recovery rates (i.e. yields) of well above 75% at the pharmaceutically relevant purification rate of at least 99.5 %.

## Claims

1. Silica particle having a fine pore size of 1 to 250 Angstrom (Å) and comprising a silane group according to the following formula: 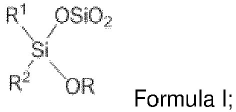
wherein R¹ and R² are each independently selected from the group consisting of alkyl, aryl, alkylaryl, heteroalkyl, heteroaryl, and heteroalkylaryl;
R is hydrogen or silica; and
SiO₂ is silica.

2. The silica particle of claim 1, wherein the fine pore size is from 50 to 150 Angstrom (Á).

3. The silica particle of claim 1 or 2, wherein R¹ and R² are the same.

4. The silica particle of any of claims 1-3, wherein R¹ and R² are alkyl, preferably C₂-C₂₀ branched or unbranched alkyl.

5. The silica particle of any of claims 1-4, wherein R¹ and R² are C₂-C₁₀ unbranched alkyl, preferably C₄-C₈ unbranched alkyl.

6. The silica particle of any of claims 1-5, wherein the silica particle has an average particle diameter of 0.1 to 100 µm, preferably from 1 to 50 µm.

7. Method for preparing a silica particle comprising the following steps:
(i) providing a silica particle with a fine pore size of 1 to 250 Angstrom (Å); and
(ii) modifying the silica particle with a compound of the following formula 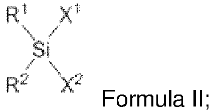
wherein R¹ and R² are each independently selected from the group consisting of alkyl, aryl, alkylaryl, heteroalkyl, heteroaryl, and heteroalkylaryl; and
X¹ and X² are each independently a halogen or an alkoxy group.

8. The method of claim 7, wherein the fine pore size is from 50 to 150 Angstrom (Á).

9. The method of claim 7 or 8, wherein R¹ and R² are the same.

10. The method of any of claims 7-9, wherein R¹ and R² are alkyl, preferably C₂-C₂₀ branched or unbranched alkyl, more preferably C₂-C₁₀ unbranched alkyl, and even more preferably C₄-C₈ unbranched alkyl.

11. The method of any of claims 7-10, wherein X¹ and X² are chlorine.

12. Silica particle obtained by the method of any of claims 7-11.

13. Separation column for chromatography comprising a silica particle of any of claims 1-6 or a silica particle obtained by the method of any of claims 7-11.

14. Use of the silica particle of any of claims 1-6 or a silica particle obtained by the method of any of claims 7-11 as stationary phase for purifying a modified conjugated peptide, preferably a GLP-1 agonist or a GLP-2 analog.

15. The use of claim 14, wherein the modified conjugated peptide is a semaglutide or liraglutide.
